# EUROPEAN PATENT SPECIFICATION

(11) **EP 4 057 896 B1**
(45) Date of publication and mention of the grant of the patent: **20.05.2026**
(21) Application number: 20888309.0
(22) Date of filing: 13.11.2020
(51) Int. Cl.: A61B 5/097, G01N 33/497

(54) **PORTABLE BREATH GAS AND VOLATILE SUBSTANCE ANALYZER**
TRAGBARES ATEMGAS UND ANALYSATOR FÜR FLÜCHTIGE SUBSTANZEN
ANALYSEUR DE GAZ ET DE SUBSTANCE VOLATILE D'HALEINE PORTATIF

(30) Priority: 14.11.2019 TH 1901007189
(43) Date of publication of application: 21.09.2022
(73) Proprietor: Health Innovision Company Limited, Bangkok, 10110 (TH)
(72) Inventor: NASOMPHAN, Weerachai, Sai Mun Yasothon, 35170 (TH)
(74) Representative: Plasseraud IP
(86) International application number: PCT/IB2020/060679
(87) International publication number: WO 2021/094986

(56) References cited:
- WO-A1-2019/074922
- US-A1- 2002 143 267
- US-A1- 2004 077 093
- US-A1- 2007 093 725
- US-A1- 2013 027 209
- US-A1- 2013 253 286
- US-A1- 2016 331 272
- US-A1- 2017 303 822

## Description

### TECHNICAL FIELD

Engineering related to a portable breath gas and volatile substance analyzer

### BACKGROUND OF THE INVENTION

Respiration is a physical activity of living organisms that happens unceasingly. In a normal state, inhalation and exhalation happen automatically as well as heart pulsation. It is apparent that respiration is a crucial process that creates equilibration in different processes within living organisms. The breath coming from respiration of living organisms is therefore an important sign that can indicate an early sign of internal imbalance or abnormality. Moreover, it has been long recognized in biomedical researches that the illnesses caused by internal abnormality in human body can cause gas molecules or volatile organic compounds (VOCs) in the respiratory system which are released along with the breath. The gas molecules or volatile organic compounds correlate with the functional status of cells, tissues, microbiome or microbes in the body which is an important indication to human health in terms of disease development.

For that reason, there are attempts to devise methods and apparatus for analyzing or monitoring disease conditions by analyzing gases and/or volatile substances present in the breath, for example, by measuring the concentration of acetone gas present in the breath which significantly correlates with the blood sugar level of diabetes patients. Such correlation can indicate diabetes and the blood sugar level without an additional blood draw step.

Researches and documents related to apparatus or methods for analyzing breath gas or volatile substance which indicate the illness identity are disclosed. Examples of the prior art are as follows.

US patent publication no. US 2013/0259748 A1 discloses a breath acetone gas sensor apparatus comprising a chamber for containing breath gas sample, an acetone gas sensor placed in said chamber for generating an output current in response to an acetone concentration of acetone gas in a breath sample, a heating device for heating the acetone gas sensor, and a measurement unit, coupled with said acetone gas sensor for providing a measured signal corresponding to said output current.

US patent publication no. US 2008/0077037 A1 discloses a medical diagnostic device for analyzing breath gases and/or skin emissions including a highly sensitive sensing component for obtaining an emission concentration profile and a database of breath analysis profiles medical condition characteristics.

US patent no. US 6341520 B1 discloses a method and apparatus for analyzing breath sample capable of analyzing the amount of gases contained in the breath by means of the coordination of chromatographic columns, a data processor, a breath sample receiving tube, a gas reservoir, and a gas valve. Said components work together automatically in the operation cycles.

Further gas analyzers are known from US-2004/077093-A1, WO-2019/074922-A1, US-2017/303822-Al, US-2007/093725-A1, US-2002/143267-Al, US-2013/253286-Al, US-2013/027209-A1, and US-2016/331272-Al.

Although the apparatus for analyzing breath gas according to the above prior arts are capable of analyzing gases, there remains a limitation as they are not specifically designed for using with the breath with low concentration of gas and high relative humidity.

### SUMMARY OF THE INVENTION

An objective of the present invention is to invent a breath gas and volatile substance analyzer that can be used and carried around conveniently with high efficiency in analyzing the gas and volatile substance. It is invented in consideration of the dynamics of the gas to be analyzed to enable the analysis of the breath gas and volatile substance even though their concentrations are extremely low, for example, at concentrations lower than 5 ppm. It is invented in consideration of the relative humidity in the breath as well.

The portable breath gas and volatile substance analyzer according to the present invention is defined in claim 1.

The portable breath gas and volatile substance analyzer according to the present invention is capable of analyzing the breath gas and volatile substance, which indicate disease characteristics which provides the technical advantages, for example,
- the analyzer can be used to conveniently and rapidly analyze illnesses or foreign substances in the body, such as addictive substances or alcohol, without causing difficulty or pain for the users as they do not need to go to the hospital for blood draw or urine collection;
- the analyzer can provide an accurate result that is comparable to the test results obtained from the blood draw or urine test which is particularly advantageous for the conditions of certain illnesses, such as diabetes wherein it is necessary to monitor the blood sugar regularly, as the breath gas analysis results obtained from the analyzer can conveniently and rapidly provide the patients with the information so that they are able to watch out for their conditions and take care of themselves without delay; and
- the analyzer can control the breath flow pattern in order to deliver gas and organic volatile substance molecules in an amount and ratio suitable to the sensor for the analysis to acquire an efficient analysis, despite the low concentration and high humidity of the gas and volatile substance molecules.

### BRIEF DESCRIPTION OF THE DRAWINGS

Figure 1 shows the components of the portable breath gas and volatile substance analyzer according to an exemplary embodiment of the present invention.
Figure 2 is a side view of the breath input portion of the portable breath gas and volatile substance analyzer according to an exemplary embodiment of the present invention.
Figure 3 is a front view of the breath input portion of the portable breath gas and volatile substance analyzer according to an exemplary embodiment of the present invention.
Figure 4 is a rear view of the breath input portion of the portable breath gas and volatile substance analyzer according to an exemplary embodiment of the present invention.
Figure 5 is a bottom view of the breath input portion of the portable breath gas and volatile substance analyzer according to an exemplary embodiment of the present invention.
Figure 6 is a cross-sectional view of the main tube of the breath flow adjustment tube of the portable breath gas and volatile substance analyzer according to an exemplary embodiment of the present invention.

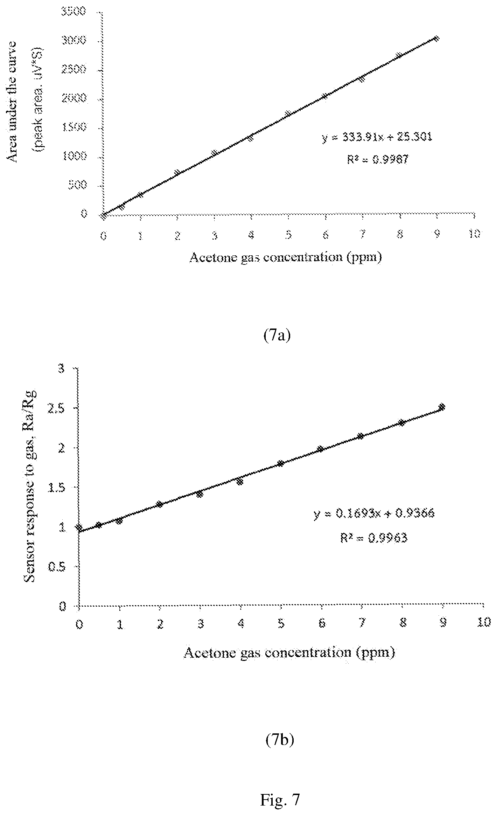

Figure 7 is a graph showing the breath acetone gas analysis results at concentrations ranging from 0 to 9 ppm, wherein (7a) shows the analysis results obtained from using the gas chromatography technique and (7b) shows the analysis results obtained from using the portable breath gas and volatile substance analyzer according to the present invention.
Figure 8 is a diagram showing changes in the electrical signals from the sensor obtained from using the portable breath gas and volatile substance analyzer according to the present invention in a clinical test for its ability to analyze the breath acetone gas from a sample group.

### DETAILED DESCRIPTION

The portable breath gas and volatile substance analyzer according to the present invention will now be described in greater detail with reference to the accompanying drawings.

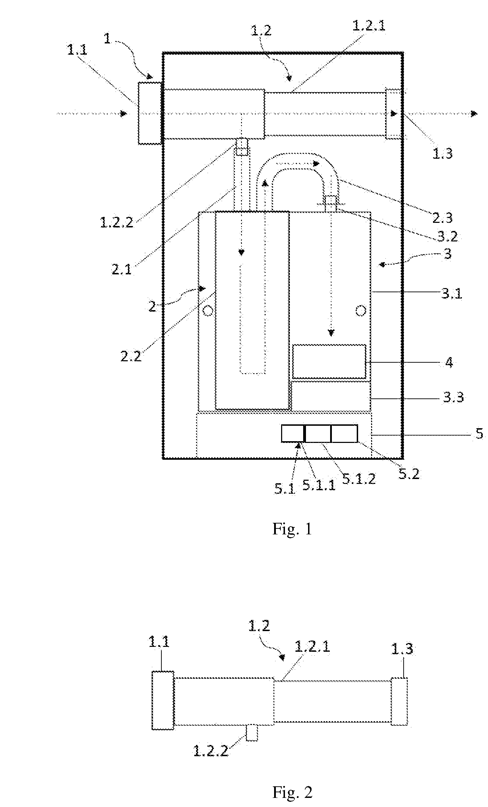

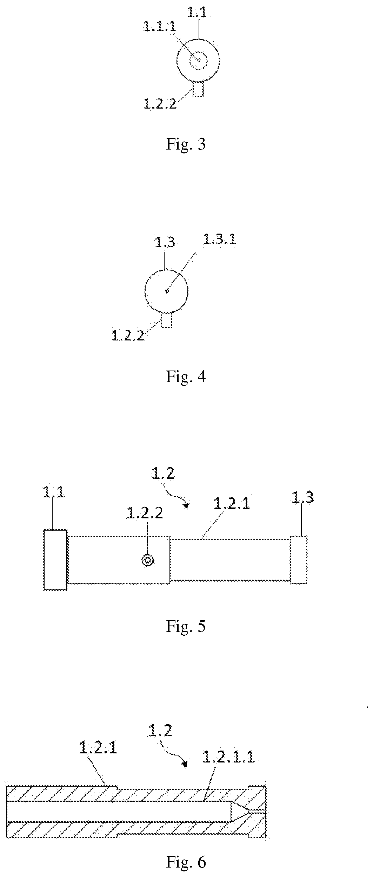

Exemplary embodiments of the portable breath gas and volatile substance analyzer according to the present invention are shown in Figures 1 to 6. The portable breath gas and volatile substance analyzer comprises the breath input portion (1) for inputting the breath into the analyzer by blowing breath, the breath delivery portion (2) connected to the breath input portion (1), the breath storage portion (3) connected to the breath delivery portion (2), the sensor (4) provided in the breath storage portion (3) for detecting the breath, and the microcontroller (5) connected to the sensor (4) for receiving the breath data from the sensor (4) to analyze the gas and volatile substance data.

According to the present invention, the breath input portion (1) comprises the mouthpiece (1.1) having the breath input channel (1.1.1) for the users to blow breath, the breath flow adjustment tube (1.2) connected to the mouthpiece (1.1), and the end portion (1.3) connected to the breath flow adjustment tube (1.2), having the breath output channel (1.3.1). The breath flow adjustment tube (1.2) comprises the main tube (1.2.1) arranged between the mouthpiece (1.1) and the end portion (1.3) of the breath input portion (1) and the breath delivery tube (1.2.2) arranged underneath the main tube (1.2.1) to serve as a channel allowing the breath to flow to the breath delivery portion (2). The breath delivery tube (1.2.2) is installed at a position 0.5 to 75 mm far from the mouthpiece (1.1) in a horizontal direction.

According to the above embodiment, the transfer of breath into the breath input portion (1) comprising the breath flow adjustment tube (1.2) with the breath delivery tube ( 1.2.2) installed in an appropriate position to deliver the breath to the breath delivery portion (2) allows the breath flow pattern to be controlled so that the gas and volatile organic substance molecules are delivered in an appropriate amount and ratio. The excess breath can be discharged through the breath output channel (1.3.1).

In a preferred embodiment, the main tube (1.2.1) is a tube having a breath flow channel (1.2.1.1) inside. The end portion of the breath flow channel (1.2.1.1) on the side which is connected to the breath output channel (1.3.1) tapers toward the breath output channel (1.3.1). Said main tube (1.2.1) has a length ranging from 15-80 mm, preferably ranging from 30-50 mm. The breath delivery tube (1.2.2) is arranged such that it is perpendicular to the horizontal axis of the main tube (1.2.1).

The breath delivery tube (1.2.2) is preferably a hollow round tube with a diameter ranging from 0.5 to 5 mm.

In an alternative embodiment, the main tube (1.2.1) and the breath delivery tube (1.2.2) are made of a material which can be selected from polyethylene, polypropylene and Teflon, preferably Teflon or polyethylene and polypropylene with a Teflon-coated inner surface. Said materials, especially Teflon, are suitable for the gas flow and corrosion-resistant. They are also more effective at eliminating contaminated gas than other materials.

According to a specific embodiment, the breath input channel (1.1.1) is an aperture with a diameter ranging from 0.5 to 15 mm and the breath output channel (1.3.1) is an aperture with a diameter ranging from 0.5 to 5 mm.

Preferably, the mouthpiece (1.1), the breath flow adjustment tube (1.2) and the end portion (1.3) are connected together in a detachable manner.

According to the present invention, the breath delivery portion (2) comprises a breath receiving tube (2.1) connected to the breath delivery tube (1.2.2) of the breath flow adjustment tube (1.2), a pump (2.2) connected to the breath receiving tube (2.1) for controlling the breath flow rate, and a breath output tube (2.3) connected to the pump (2.2) for transferring the breath to the breath storage portion (3) at a region near the sensor (4).

The pump (2.2) is preferably installed separately close to the breath storage portion (3). The pump (2.2) controls the breath flow rate to be within a range of 0 to 3 L/min.

According to the above embodiment, the breath flow is controlled by the pump (2.2) so that the flow is constant and hits the region near the sensor (4) with a speed suitable for the sensor response, which is not too fast or too slow, therefore enabling the sensor to detect the gases and volatile substances more efficiently.

The pump (2.2) may be operated intermittently or continuously. The intermittent operation period takes 0.06 to 1 minute and the continuous operation period takes 0.06 to 10 minutes.

Alternatively, the breath receiving tube (2.1) and the breath output tube (2.3) are made of silicone or Teflon.

According to the present invention, the breath storage portion (3) comprises a storage chamber (3.1), an inlet (3.2) arranged above the storage chamber (3.1) to serve as a channel for inputting the breath received from the breath delivery portion (2) and a moisture discharge port (3.3) arranged underneath the storage chamber (3.1).

According to a preferred embodiment, the storage chamber (3.1) has a volume ranging from 0.10 to 3 liters and has a cylindrical shape with a diameter ranging from 15 to 35 mm and a height ranging from 30 to 70 mm.

By placing the inlet (3.2) above the storage chamber (3.1) and the sensor (4) underneath the storage chamber (3.1) at a suitable height, the breath flowing into the storage chamber (3.1) will not contact with the sensor (4) immediately; instead, it will gradually flow into the storage chamber (3.1) with a controlled speed and diffuse to properly contact with the sensor (4), allowing the sensor to respond to the gas and volatile substance more effectively. In addition, the moisture discharge port (3.3) also helps to discharge the gas or moisture remaining in the storage chamber (3.1) and prevent condensation of water vapor in the system.

In one embodiment, the sensor (4) may be an acetone gas-specific metal oxide sensor which makes the breath gas and volatile substance analyzer particularly suitable for use to indicate diabetes and the blood sugar level. In an alternative aspect, the sensor (4) may be a temperature sensor, moisture sensor or a combination thereof to help enhance the gas or volatile substance analytical efficiency in terms of both quality and quantity.

According to the present invention, the microcontroller (5) comprises a processor (5.1) for processing the breath data obtained from the sensor (4) and a controller (5.2) for controlling the operation of the breath delivery portion (2). The processor (5.1) comprises a feature generating portion (5.1.1) for generating features from the breath data signal detected by the sensor (4) and a computing portion (5.1.2) for processing the feature to obtain the illness identity.

As an example, the feature generating portion (5.1.1) generates features from the voltage data signal of the sensor (4) and the computing portion (5.1.2) is operated using the artificial neural networks and/or artificial intelligence method.

To use the portable breath gas and volatile substance analyzer, the users blow their breath through the breath input portion (1). The breath then flows into the main tube (1.2.1) (shown as a dashed line in Figure 1) wherein a portion of the breath to be analyzed continues to flow to the breath delivery tube (1.2.2) and another portion which is an excess breath is discharged through the breath output channel (1.3.1).

The breath to be analyzed flows from the breath delivery tube (1.2.2) into the breath receiving tube (2.1) and is transferred to the breath output tube (2.3). The flow rate is controlled by the pump (2.2).

The breath from the breath output tube (2.3) is further transferred to the storage chamber (3.1) in the upper part above the sensor (4) and then hits the sensor (4), which responds specifically to the target gas or volatile substance molecules.

The signal received from the sensor (4) which is specific to the target gas and volatile substance molecules in the breath is transferred to the microcontroller (5) and analyzed to identify the features. Suitable features are then selected for the analysis of the amount of target gas or volatile substance molecules. A predictive model is then created using the artificial neural networks and/or artificial intelligence. Said predictive model is used to calculate the amount of target gas and volatile substance molecules in the breath.

In an additional embodiment, the portable breath gas and volatile substance analyzer according to the present invention may further comprise a display (6) (not shown in the drawings) for displaying the results of gases and volatile substances analysis. The display (6) may be a display with or without a display monitor.

Moreover, the portable breath gas and volatile substance analyzer according to the present invention may further comprise a housing (7) (not shown in the drawings) provided for covering the portable breath gas and volatile substance analyzer such that the mouthpiece (1.1) of the breath input portion (1) extends outwardly. The housing (7) is provided to prevent the internal components from exposing to an environment and to enhance the aesthetics of the analyzer.

The test results from using the portable breath gas and volatile substance analyzer according to the present invention compared with the result of the analysis using a standard technique will now be described.

The test used the acetone gas as a representative gas in the test by simulating the condition of the breath acetone gas, that is, the acetone gas with a low concentration ranging from 0 to 9 ppm. The response of the sensor (4) of the analyzer according to the present invention to the gas is measured in comparison with the response to the gas measured using a standard technique, i.e. gas chromatography technique.

The breath acetone gas analysis result obtained by using the gas chromatography technique is shown as the area under the curve compared with the concentration of the acetone gas as shown in Figure (7a). The analysis result obtained from using the analyzer according to the present invention is shown as the sensor response to the gas compared with the concentration of the acetone gas as shown in Figure (7b).

According to the analysis results shown in Figures (7a) and (7b), it was found that the response to the acetone gas with a concentration of 9 ppm was at the highest level and that the sensor response to the acetone gas decreased in accordance with a decline of gas concentration. In summary, the response to gas varies directly with the concentration of acetone gas which corresponds to the analysis result obtained by using the gas chromatography technique and the lowest acetone gas concentration which causes a response is 0.5 ppm. This shows that the analyzer according to the present invention can be used to analyze the acetone gas with a concentration as low as 0.5 ppm.

The result of acetone gas analysis which was conducted using the gas and volatile substance analyzer according to the present invention compared with the result obtained using the gas chromatography technique and the data from the graphs in Figures (7a) and (7b) are interpreted into method validation parameters as shown below in Table 1.

**Table 1**

| **Method validation parameter** | **Analysis using the analyzer according to the present invention** | **Analysis using the gas chromatography technique** |
|---|---|---|
| Maximum detectability | Gas response = 2.482 | Area under the curve = 3012.7 |
| Acetone gas concentration range | 0 to 9 ppm | 0 to 9 ppm |
| Linear equation (Regression equation, y = mx+c) | y = 0.1693x + 0.9366 | y = 333.91x + 25.301 |
| Slope | 0.1693 | 333.91 |
| Y intercept | 0.9366 | 25.301 |
| Coefficient of correlation | 0.9963 | 0.9987 |
| Repeatability accuracy, %RSD (n=3) | 0.282 to 1.664 | 0.355 to 7.311 |
| Analysis accuracy, %RSD | 87.1 to 101.8 | 71.3 to 106.2 |
| Intra-day analysis precision, %RSD | 1.211 | 7.311 |
| Inter-day analysis precision, %RSD | 1.872 | 0.055 |

The parameters shown in Table 1 above demonstrate that the acetone gas analysis using the analyzer according to the present invention provides recovery values in a range of 80-120% of the analysis using the analysis using the standard gas chromatography technique which corresponds to the criteria for assessing the features of a suitable analysis method according to the Association of Official Agricultural Chemists International standard.

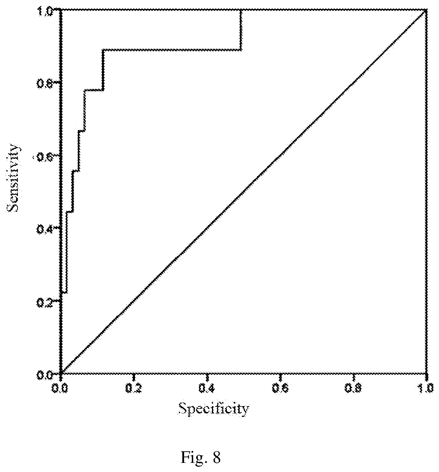

Moreover, a clinical test was also carried out to test the performance of the analyzer according to the present invention which was used with a group of 76 people, which was divided into a control sample group and a sample group with diabetic ketoacidosis (DKA). The analyzer according to the present invention was used to measure the amount of breath acetone gas from the sample group. The response to electrical signal was then analyzed and calculated to create a diagram reflecting a change in the electrical signal as shown in Figure 8. The summary of the signal interpretation is provided below in Table 2.

**Table 2**

| **Parameters from the clinical test** | **Electrical signal interpretation results** |
|---|---|
| Optimal intercept of sensor response | 1.602 |
| Sensitivity | 88.9% |
| Specificity | 88.5% |
| P value | < 0.001 |
| Area under the curve (AUC) | 0.931 |
| 95% CI | 0.809; 1.016 |

It can be seen that a change in the electrical signal obtained from the analyzer according to the present invention corresponds to the breath acetone gas level. There is a statistically significant difference (P value < 0.001) between the breath acetone level of the sample group with DKA and the breath acetone level of the control sample group. The receiver operating characteristic (ROC) curve can be plotted to select an optimal intercept for the sensitivity and specificity interpretation from the clinical test of the analyzer according to the present invention.

The portable breath gas and volatile substance analyzer according to the present invention is not limited to the embodiments described above and the drawings. Any modifications or changes may be made, for example, the portable breath gas and volatile substance analyzer according to the present invention may be modified to be able to detect the identity of other illnesses other than diabetes by adjusting the design or type of the sensor (4). Modifications or changes with the aim of measuring the amount of volatile substance or gas to indicate addictive substances or alcohol in the blood, for example, may also be made. Such modifications or changes are still considered to be within the scope of the present invention.

### BEST MODE OF THE INVENTION

Best mode of the invention is as described in the detailed description of the invention.

## Claims

1. A portable breath gas and volatile substance analyzer comprising
a breath input portion (1) for inputting the breath into the analyzer by blowing breath,
a breath delivery portion (2) connected to the breath input portion (1),
a breath storage portion (3) connected to the breath delivery portion (2),
a sensor (4) provided in the breath storage portion (3) for detecting the breath, and
a microcontroller (5) connected to the sensor (4) for receiving the breath data from the sensor (4) to analyze the gas and volatile substance data,
wherein
the breath input portion (1) comprises a mouthpiece (1.1) having a breath input channel (1.1.1) for the users to blow breath, a breath flow adjustment tube (1.2) connected to the mouthpiece (1.1), and an end portion (1.3) connected to the breath flow adjustment tube (1.2), having a breath output channel (1.3.1)
wherein the breath flow adjustment tube (1.2) comprises a main tube (1.2.1) arranged between the mouthpiece (1.1) and the end portion (1.3) of the breath input portion (1), and a breath delivery tube (1.2.2) arranged underneath the main tube (1.2.1) to serve as a channel allowing the breath to continue to flow to the breath delivery portion (2), the breath delivery tube (1.2.2) being installed at a position 0.5 to 75 mm far from the mouthpiece (1.1) in a horizontal direction,
the breath storage portion (3) comprises a storage chamber (3.1), an inlet (3.2) arranged above the storage chamber (3.1) to serve as a channel for inputting the breath received from the breath delivery portion (2) and a moisture discharge port (3.3) arranged underneath the storage chamber (3.1),
the sensor (4) is positioned underneath the storage chamber (3.1), and the storage chamber (3.1) has a height ranging from 30 to 70 mm.

2. The portable breath gas and volatile substance analyzer according to claim 1, wherein the main tube (1.2.1) is a tube having a breath flow channel (1.2.1.1) on the inner side, the end portion of the breath flow channel (1.2.1.1) on the side which is connected to the breath output channel (1.3.1) being tapered toward the breath output channel (1.3.1).

3. The portable breath gas and volatile substance analyzer according to claim 1, wherein the main tube (1.2.1) has a length in a range of 15-80 mm, preferably in a range of 30-50 mm.

4. The portable breath gas and volatile substance analyzer according to claim 1, wherein the breath delivery tube (1.2.2) is arranged such that it is perpendicular to the horizontal axis of the main tube (1.2.1).

5. The portable breath gas and volatile substance analyzer according to claim 1 or 4, wherein the breath delivery tube (1.2.2) is a hollow round tube with a diameter ranging from 0.5 to 5 mm.

6. The portable breath gas and volatile substance analyzer according to any one of claims 1 to 5, wherein the main tube (1.2.1) and the breath delivery tube (1.2.2) are made of a material which is selected from polyethylene, polypropylene and Teflon, preferably Teflon or polyethylene and polypropylene with a Teflon-coated inner surface.

7. The portable breath gas and volatile substance analyzer according to claim 1, wherein the breath input channel (1.1.1) is an aperture with a diameter ranging from 0.5 to 15 mm and the breath output channel (1.3.1) is an aperture with a diameter ranging from 0.5 to 5 mm.

8. The portable breath gas and volatile substance analyzer according to claim 1, wherein the mouthpiece (1.1), the breath flow adjustment tube (1.2) and the end portion (1.3) are connected together in a detachable manner.

9. The portable breath gas and volatile substance analyzer according to claim 1, wherein the breath delivery portion (2) comprises a breath receiving tube (2.1) connected to the breath delivery tube ( 1.2.2) of the breath flow adjustment tube (1.2), a pump (2.2) connected to the breath receiving tube (2.1) for controlling the breath flow rate, and the breath output tube (2.3) connected to the pump (2.2) for transferring the breath to the breath storage portion (3) at a region near the sensor (4).

10. The portable breath gas and volatile substance analyzer according to claim 9, wherein the pump (2.2) is installed separately close to the breath storage portion (3).

11. The portable breath gas and volatile substance analyzer according to claim 9 or 10, wherein the pump (2.2) controls the breath flow rate to be within a range of 0 to 3 L/min.

12. The portable breath gas and volatile substance analyzer according to any one of claims 9 to 11, wherein the pump (2.2) is operated intermittently or continuously with the intermittent operation period being 0.06 to 1 minute and the continuous operation period being 0.06 to 10 minutes.

13. The portable breath gas and volatile substance analyzer according to claim 9, wherein the breath receiving tube (2.1) and the breath output tube (2.3) are made of a material which is silicone or Teflon.

14. The portable breath gas and volatile substance analyzer according to claim 1, wherein the storage chamber (3.1) has a volume ranging from 0.10 to 3 liters.

15. The portable breath gas and volatile substance analyzer according to claim 1 or 14, wherein the storage chamber (3.1) has a cylindrical shape with a diameter ranging from 15 to 35 mm.

16. The portable breath gas and volatile substance analyzer according to claim 1, wherein the sensor (4) is an acetone gas-specific metal oxide sensor.

17. The portable breath gas and volatile substance analyzer according to claim 1, wherein the sensor (4) is a temperature sensor, moisture sensor or a combination thereof.

18. The portable breath gas and volatile substance analyzer according to claim 1 wherein, the microcontroller (5) comprises a processor (5.1) for processing the breath data obtained from the sensor (4) and a controller (5.2) for controlling the operation of the breath delivery portion (2).

19. The portable breath gas and volatile substance analyzer according to claim 18, wherein the processor (5.1) comprises a feature generating portion (5.1.1) for generating features from the breath data signal detected by the sensor (4) and a computing portion (5.1.2) for processing the features to obtain the values indicating the illness identity.

20. The portable breath gas and volatile substance analyzer according to claim 19, wherein the feature generating portion (5.1.1) generates features from the voltage data signal of the sensor (4).

21. The portable breath gas and volatile substance analyzer according to claim 19, wherein the computing portion (5.1.2) is operated using the artificial neural networks and/or artificial intelligence method.

22. The portable breath gas and volatile substance analyzer according to any one of claims 1 to 21 further comprising a display (6) for displaying the results of gases and volatile substances analysis.

23. The portable breath gas and volatile substance analyzer according to claim 22, wherein the display (6) is a display with or without a monitor.

24. The portable breath gas and volatile substance analyzer according to any one of claims 1 to 23 further comprising a housing (7) for covering the portable breath gas and volatile substance analyzer such that the mouthpiece (1.1) of the breath input portion (1) extends outwardly.

## Patentansprüche

1. Tragbares Analysegerät für Atemgase und flüchtige Substanzen, umfassend einen Atemeinlassabschnitt (1) zum Einlassen des Atems in das Analysegerät durch Blasen von Atem,
einen Atemabgabeabschnitt (2), der mit dem Atemeinlassabschnitt (1) verbunden ist, einen Atemspeicherabschnitt (3), der mit dem Atemabgabeabschnitt (2) verbunden ist, einen Sensor (4), der in dem Atemspeicherabschnitt (3) zum Erfassen des Atems bereitgestellt ist, und
einen mit dem Sensor (4) verbundenen Mikrocontroller (5) zum Empfangen der Atemdaten von dem Sensor (4), um die Daten für Gas und flüchtige Substanzdaten zu analysieren,
wobei
der Atemeinlassabschnitt (1) ein Mundstück (1.1) umfasst, das einen Atemeinlasskanal (1.1.1) für die Benutzer, um Atem zu blasen, einen mit dem Mundstück (1.1) verbundenen Atemstrom-Einstellschlauch (1.2) und einen mit dem Atemstrom-Einstellschlauch (1.2) verbundenen Endabschnitt (1.3) aufweist, der einen Atemauslasskanal (1.3.1) aufweist,
wobei der Atemstrom-Einstellschlauch (1.2) einen Hauptschlauch (1.2.1) umfasst, der zwischen dem Mundstück (1.1) und dem Endabschnitt (1.3) des Atemeinlassabschnitts (1) angeordnet ist, und einen Atemabgabeschlauch (1.2.2), der unterhalb des Hauptschlauchs (1.2.1) angeordnet ist, um als Kanal zu dienen, der es dem Atem ermöglicht, weiter zum Atemabgabeabschnitt (2) zu strömen, wobei der Atemabgabeschlauch (1.2.2) an einer Position 0,5 bis 75 mm entfernt von dem Mundstück (1.1) in horizontaler Richtung installiert ist,
der Atemspeicherabschnitt (3) eine Speicherkammer (3.1), einen Eingang (3.2), der oberhalb der Speicherkammer (3.1) angeordnet ist, um als Kanal für den Einlass des von dem Atemabgabeabschnitt (2) empfangenen Atems zu dienen, und einen Feuchtigkeitsablaufanschluss (3.3), der unterhalb der Speicherkammer (3.1) angeordnet ist, umfasst,
der Sensor (4) unterhalb der Speicherkammer (3,1) positioniert ist und die Speicherkammer (3,1) eine Höhe im Bereich von 30 bis 70 mm aufweist.

2. Tragbares Analysegerät für Atemgase und flüchtige Substanzen nach Anspruch 1, wobei der Hauptschlauch (1.2.1) ein Schlauch ist, der an der Innenseite einen Atemstromkanal (1.2.1.1) aufweist, wobei der Endabschnitt des Atemstromkanals (1.2.1.1) auf der Seite, die mit dem Atemauslasskanal (1.3.1) verbunden ist, in Richtung des Atemauslasskanals (1.3.1) schmal zulaufend ist.

3. Tragbares Analysegerät für Atemgase und flüchtige Substanzen nach Anspruch 1, wobei der Hauptschlauch (1.2.1) eine Länge in einem Bereich von 15-80 mm, vorzugsweise in einem Bereich von 30-50 mm aufweist.

4. Tragbares Analysegerät für Atemgase und flüchtige Substanzen nach Anspruch 1, wobei der Atemabgabeschlauch (1.2.2) angeordnet ist, sodass er senkrecht zur horizontalen Achse des Hauptschlauchs (1.2.1) steht.

5. Tragbares Analysegerät für Atemgase und flüchtige Substanzen nach Anspruch 1 oder 4, wobei der Atemabgabeschlauch (1.2.2) ein hohler runder Schlauch mit einem Durchmesser im Bereich von 0,5 bis 5 mm ist.

6. Tragbares Analysegerät für Atemgase und flüchtige Substanzen nach einem der Ansprüche 1 bis 5, wobei der Hauptschlauch (1.2.1) und der Atemabgabeschlauch (1.2.2) aus einem Material gefertigt sind, das aus Polyethylen, Polypropylen und Teflon, vorzugsweise Teflon oder Polyethylen und Polypropylen mit einer teflonbeschichteten Innenoberfläche ausgewählt ist.

7. Tragbares Analysegerät für Atemgase und flüchtige Substanzen nach Anspruch 1, wobei der Atemeinlasskanal (1.1.1) eine Öffnung mit einem Durchmesser im Bereich von 0,5 bis 15 mm, und der Atemauslasskanal (1.3.1) eine Öffnung mit einem Durchmesser im Bereich von 0,5 bis 5 mm ist.

8. Tragbares Analysegerät für Atemgase und flüchtige Substanzen nach Anspruch 1, wobei das Mundstück (1.1), der Atemstrom-Einstellschlauch (1.2) und der Endabschnitt (1.3) lösbar miteinander verbunden sind.

9. Tragbares Analysegerät für Atemgase und flüchtige Substanzen nach Anspruch 1, wobei der Atemabgabeabschnitt (2) einen Atemaufnahmeschlauch (2.1), der mit dem Atemabgabeschlauch (1.2.2) des Atemstrom-Einstellschlauchs (1.2) verbunden ist, eine Pumpe (2.2), die mit dem Atemaufnahmeschlauch (2.1) zur Steuerung der Atemstromrate verbunden ist, und den Atemausgangsschlauch (2.3), der zum Übertragen des Atems in den Atemspeicherabschnitt (3) in einem Bereich in der Nähe des Sensors (4) mit der Pumpe (2.2) verbunden ist, umfasst.

10. Tragbares Analysegerät für Atemgase und flüchtige Substanzen nach Anspruch 9, wobei die Pumpe (2.2) separat unweit des Atemspeicherabschnitts (3) installiert ist.

11. Tragbares Analysegerät für Atemgase und flüchtige Substanzen nach Anspruch 9 oder 10, wobei die Pumpe (2.2) die Atemstromrate in einem Bereich von 0 bis 3 l/min steuert.

12. Tragbares Analysegerät für Atemgase und flüchtige Substanzen nach einem der Ansprüche 9 bis 11, wobei die Pumpe (2.2) intermittierend oder kontinuierlich betrieben wird, wobei die intermittierende Betriebsdauer 0,06 bis 1 Minute, und die kontinuierliche Betriebsdauer 0,06 bis 10 Minuten beträgt.

13. Tragbares Analysegerät für Atemgase und flüchtige Substanzen nach Anspruch 9, wobei der Atemaufnahmeschlauch (2,1) und der Atemausgangsschlauch (2.3) aus einem Material gefertigt sind, das Silikon oder Teflon ist.

14. Tragbares Analysegerät für Atemgase und flüchtige Substanzen nach Anspruch 1, wobei die Speicherkammer (3.1) ein Volumen im Bereich von 0,10 bis 3 Liter aufweist.

15. Tragbares Analysegerät für Atemgase und flüchtige Substanzen nach Anspruch 1 oder 14, wobei die Speicherkammer (3.1) eine zylindrische Form mit einem Durchmesser im Bereich von 15 bis 35 mm aufweist.

16. Tragbares Analysegerät für Atemgase und flüchtige Substanzen nach Anspruch 1, wobei der Sensor (4) ein für Acetongas spezifischer Metalloxidsensor ist.

17. Tragbares Analysegerät für Atemgase und flüchtige Substanzen nach Anspruch 1, wobei der Sensor (4) ein Temperatursensor, Feuchtesensor oder eine Kombination davon ist.

18. Tragbares Analysegerät für Atemgase und flüchtige Substanzen nach Anspruch 1, wobei der Mikrocontroller (5) einen Prozessor (5.1) zum Verarbeiten der von dem Sensor (4) erhaltenen Atemdaten und einen Controller (5.2) zum Steuern des Betriebs des Atemabgabeabschnitts (2) umfasst.

19. Tragbares Analysegerät für Atemgase und flüchtige Substanzen nach Anspruch 18, wobei der Prozessor (5.1) einen Merkmalserzeugungsabschnitt (5.1.1) zum Erzeugen von Merkmalen aus dem von dem Sensor (4) erfassten Atemdatensignal und einen Rechenabschnitt (5.1.2) zum Verarbeiten der Merkmale umfasst, um die Werte zu erhalten, welche die Krankheitsidentität angeben.

20. Tragbares Analysegerät für Atemgase und flüchtige Substanzen nach Anspruch 19, wobei der Merkmalserzeugungsabschnitt (5.1.1) Merkmale aus dem Spannungsdatensignal des Sensors (4) erzeugt.

21. Tragbares Analysegerät für Atemgase und flüchtige Substanzen nach Anspruch 19, wobei der Rechenabschnitt (5.1.2) unter Verwendung des künstlichen neuronalen Netzwerks und/oder künstlichen Intelligenzverfahrens betrieben wird.

22. Tragbares Analysegerät für Atemgase und flüchtige Substanzen nach einem der Ansprüche 1 bis 21, weiter umfassend eine Anzeige (6) zum Anzeigen der Ergebnisse der Analyse für Gase und flüchtige Substanzen.

23. Tragbares Analysegerät für Atemgase und flüchtige Substanzen nach Anspruch 22, wobei die Anzeige (6) eine Anzeige mit oder ohne Monitor ist.

24. Tragbares Analysegerät für Atemgase und flüchtige Substanzen nach einem der Ansprüche 1 bis 23, weiter umfassend ein Gehäuse (7) zum Abdecken des tragbaren Analysegeräts für Atemgase und flüchtige Substanzen, sodass sich das Mundstück (1.1) des Atemeinlassabschnitts (1) nach außen erstreckt.

## Revendications

1. Analyseur de gaz et de substances volatiles d'haleine portatif comprenant une partie d'entrée d'haleine (1) pour entrer l'haleine dans l'analyseur par insufflation d'haleine,
une partie de délivrance d'haleine (2) reliée à la partie d'entrée d'haleine (1),
une partie de stockage d'haleine (3) reliée à la partie de délivrance d'haleine (2),
un capteur (4) prévu dans la partie de stockage d'haleine (3) pour détecter l'haleine, et un microcontrôleur (5) relié au capteur (4) pour recevoir les données d'haleine du capteur (4) pour analyser les données de gaz et de substances volatiles,
dans lequel
la partie d'entrée d'haleine (1) comprend un embout buccal (1.1) présentant un canal d'entrée d'haleine (1.1.1) pour que les utilisateurs insufflent de l'haleine, un tube d'ajustement de débit d'haleine (1.2) relié à l'embout buccal (1.1), et une partie d'extrémité (1.3) reliée au tube d'ajustement de débit d'haleine (1.2), présentant un canal de sortie d'haleine (1.3.1)
dans lequel le tube d'ajustement de débit d'haleine (1.2) comprend un tube principal (1.2.1) agencé entre l'embout buccal (1.1) et la partie d'extrémité (1.3) de la partie d'entrée d'haleine (1), et un tube de délivrance d'haleine (1.2.2) agencé sous le tube principal (1.2.1) pour servir de canal permettant à l'haleine de continuer à s'écouler vers la partie de délivrance d'haleine (2), le tube de délivrance d'haleine (1.2.2) étant installé à une position éloignée de 0,5 à 75 mm de l'embout buccal (1.1) dans une direction horizontale,
la partie de stockage d'haleine (3) comprend une chambre de stockage (3.1), une entrée (3.2) agencée au-dessus de la chambre de stockage (3.1) pour servir de canal pour entrer l'haleine reçue de la partie de délivrance d'haleine (2) et un orifice d'évacuation d'humidité (3.3) agencé sous la chambre de stockage (3.1),
le capteur (4) est positionné sous la chambre de stockage (3.1), et la chambre de stockage (3.1) présente une hauteur allant de 30 à 70 mm.

2. Analyseur de gaz et de substances volatiles d'haleine portatif selon la revendication 1, dans lequel le tube principal (1.2.1) est un tube présentant un canal d'écoulement d'haleine (1.2.1.1) sur le côté intérieur, la partie d'extrémité du canal d'écoulement d'haleine (1.2.1.1) sur le côté qui est relié au canal de sortie d'haleine (1.3.1) étant effilée vers le canal de sortie d'haleine (1.3.1).

3. Analyseur de gaz et de substances volatiles d'haleine portatif selon la revendication 1, dans lequel le tube principal (1.2.1) présente une longueur dans une plage de 15-80 mm, de préférence dans une plage de 30-50 mm.

4. Analyseur de gaz et de substances volatiles d'haleine portatif selon la revendication 1, dans lequel le tube de délivrance d'haleine (1.2.2) est agencé de sorte qu'il soit perpendiculaire à l'axe horizontal du tube principal (1.2.1).

5. Analyseur de gaz et de substances volatiles d'haleine portatif selon la revendication 1 ou 4, dans lequel le tube de délivrance d'haleine (1.2.2) est un tube rond creux avec un diamètre allant de 0,5 à 5 mm.

6. Analyseur de gaz et de substances volatiles d'haleine portatif selon l'une quelconque des revendications 1 à 5, dans lequel le tube principal (1.2.1) et le tube de délivrance d'haleine (1.2.2) sont fabriqués dans un matériau qui est choisi parmi le polyéthylène, le polypropylène et le Téflon, de préférence le Téflon ou le polyéthylène et le polypropylène avec une surface interne revêtue de Téflon.

7. Analyseur de gaz et de substances volatiles d'haleine portatif selon la revendication 1, dans lequel le canal d'entrée d'haleine (1.1.1) est une ouverture avec un diamètre allant de 0,5 à 15 mm et le canal de sortie d'haleine (1.3.1) est une ouverture avec un diamètre allant de 0,5 à 5 mm.

8. Analyseur de gaz et de substances volatiles d'haleine portatif selon la revendication 1, dans lequel l'embout buccal (1.1), le tube d'ajustement de débit d'haleine (1.2) et la partie d'extrémité (1.3) sont reliés ensemble de manière détachable.

9. Analyseur de gaz et de substances volatiles d'haleine portatif selon la revendication 1, dans lequel la partie de délivrance d'haleine (2) comprend un tube de réception d'haleine (2.1) relié au tube de délivrance d'haleine (1.2.2) du tube d'ajustement de débit d'haleine (1.2), une pompe (2.2) reliée au tube de réception d'haleine (2.1) pour contrôler le débit d'haleine, et le tube de sortie d'haleine (2.3) relié à la pompe (2.2) pour transférer l'haleine vers la partie de stockage d'haleine (3) au niveau d'une région proche du capteur (4).

10. Analyseur de gaz et de substances volatiles d'haleine portatif selon la revendication 9, dans lequel la pompe (2.2) est installée séparément à proximité de la partie de stockage d'haleine (3).

11. Analyseur de gaz et de substances volatiles d'haleine portatif selon la revendication 9 ou 10, dans lequel la pompe (2.2) contrôle le débit d'haleine pour qu'il soit dans une plage de 0 à 3 l/min.

12. Analyseur de gaz et de substances volatiles d'haleine portatif selon l'une quelconque des revendications 9 à 11, dans lequel la pompe (2.2) fonctionne de manière intermittente ou continue, la période de fonctionnement intermittent étant de 0,06 à 1 minute et la période de fonctionnement continu étant de 0,06 à 10 minutes.

13. Analyseur de gaz et de substances volatiles d'haleine portatif selon la revendication 9, dans lequel le tube de réception d'haleine (2.1) et le tube de sortie d'haleine (2.3) sont fabriqués dans un matériau qui est du silicone ou du Téflon.

14. Analyseur de gaz et de substances volatiles d'haleine portatif selon la revendication 1, dans lequel la chambre de stockage (3.1) présente un volume allant de 0,10 à 3 litres.

15. Analyseur de gaz et de substances volatiles d'haleine portatif selon la revendication 1 ou 14, dans lequel la chambre de stockage (3.1) présente une forme cylindrique avec un diamètre allant de 15 à 35 mm.

16. Analyseur de gaz et de substances volatiles d'haleine portatif selon la revendication 1, dans lequel le capteur (4) est un capteur d'oxyde métallique spécifique au gaz acétone.

17. Analyseur de gaz et de substances volatiles d'haleine portatif selon la revendication 1, dans lequel le capteur (4) est un capteur de température, un capteur d'humidité ou une combinaison de ceux-ci.

18. Analyseur de gaz et de substances volatiles d'haleine portatif selon la revendication 1, dans lequel le microcontrôleur (5) comprend un processeur (5.1) pour traiter les données d'haleines obtenues à partir du capteur (4) et un contrôleur (5.2) pour contrôler le fonctionnement de la partie de délivrance d'haleine (2).

19. Analyseur de gaz et de substances volatiles d'haleine portatif selon la revendication 18, dans lequel le processeur (5.1) comprend une partie de génération de caractéristiques (5.1.1) pour générer des caractéristiques à partir du signal de données d'haleine détecté par le capteur (4) et une partie de calcul (5.1.2) pour traiter les caractéristiques pour obtenir les valeurs indiquant l'identité de la maladie.

20. Analyseur de gaz et de substances volatiles d'haleine portatif selon la revendication 19, dans lequel la partie génératrice de caractéristiques (5.1.1) génère des caractéristiques à partir du signal de données de tension du capteur (4).

21. Analyseur de gaz et de substances volatiles d'haleine portatif selon la revendication 19, dans lequel la partie de calcul (5.1.2) fonctionne à l'aide des réseaux neuronaux artificiels et/ou du procédé d'intelligence artificielle.

22. Analyseur de gaz et de substances volatiles d'haleine portatif selon l'une quelconque des revendications 1 à 21 comprenant en outre un affichage (6) pour afficher les résultats d'analyse de gaz et de substances volatiles.

23. Analyseur de gaz et de substances volatiles d'haleine portatif selon la revendication 22, dans lequel l'affichage (6) est un affichage avec ou sans moniteur.

24. Analyseur de gaz et de substances volatiles d'haleine portatif selon l'une quelconque des revendications 1 à 23 comprenant en outre un boîtier (7) pour recouvrir l'analyseur de gaz et de substances volatiles d'haleine portatif de sorte que l'embout buccal (1.1) de la partie d'entrée d'haleine (1) s'étend vers l'extérieur.
